# EUROPEAN PATENT APPLICATION

(11) **EP 1 138 338 A1**
(43) Date of publication of application: **04.10.2001**
(21) Application number: 01103024.4
(22) Date of filing: 09.02.2001
(51) Int. Cl.: A61M 5/32, A61M 5/00

(54) **Medical safety disposal cap assembly**

(30) Priority: 20.03.2000 US 528313
(71) Applicant: Becton, Dickinson and Company, Franklin Lakes, New Jersey 07417 (US)
(72) Inventor: Chelak, Todd M., Waldwick, NJ 07463 (US); Di Biasi, Michael A., West Milford, NJ 07480 (US); Nguyen,Tuan V., No. Plainfield, NJ 07060 (US); Sharifi-Mehr, Amir Ali, Springfield, NJ 07081 (US); West, Robert E., Basking Ridge, NJ 07920 (US)
(74) Representative: von Kreisler, Alek, Dipl.-Chem.

(57) **Abstract**

A safety disposal cap for use with pen needles allows patients to safely dispose of needles without having to carry a sharps container. The cap seals the outer cover over the patient end of the pen needle while providing protection also to the non-patient end of the needle. The cap can be made as a unitary structure with the outer cover or as a separate component.

## Description

### FIELD OF THE INVENTION

This invention generally relates to a safety device for medical products, more particularly, this invention relates to a safety disposal cap for pen needles.

### BACKGROUND OF THE INVENTION

In the medical art, the current usage of pen needles involves a patient removing a paper label from the needle to expose a non-patient end of the needle and inserting the non-patient end of the needle onto an injection pen containing a medication cartridge. After use, the needle hub is disassembled from the device and is usually thrown into a sharps container. This procedure describes the usual method of disposing pen needles, and although it provides a safe and easy way of disposing pen needles, it does not provide a convenient method for the user since the user has to carry a sharps container during use of the medication delivery device. There is usually a gap in time between the use of the injection pen and the disposal of the pen needle.

In the prior art, as seen in U.S. Patent 5,971,966 to Lav, this time gap can be shortened by using a magazine for the storage and final disposal of the pen needle by a snap-on needle unit. However, this device leaves exposed the non-patient end (hereinafter "NP end") and potentially has exposed a medical sharp containing blood borne pathogens. In the current medical arts, there is a great need to avoid exposure to blood borne pathogens due to various diseases that can result in the exposure to such pathogens. Thus, this prior art device does not provide disposal and protection of the NP end of the needle. In addition, it enhances a sense of false security in safely disposing the pen needle by only securing the patient end of the pen needle.

Additional attempts in the prior art to provide a safety disposal cap may be seen in U.S. Patent 5,968,021 to Ejlersen. This device provides a needle unit with a needle mounted onto the hub. The needle unit has a sleeve made of a deformable material such that the sleeve may snap lock onto a connecting piece of the pen needle. As with the previous prior art, this medical device provides protection from the patient end of the needle, however, it exposes the non-patient end to inadvertent needle sticks. Therefore, there still exists a need in the art which allows simple and easy disposure of the pen needle directly after use. The device should contain an assembly that provides protection to both the patient and NP end of the pen needle.

### SUMMARY OF THE INVENTION

The present invention avoids the disadvantages of the prior art by providing a safety disposal cap assembly that will protect both the patient and the non-patient end after the pen needle is used. The safety disposal cap assembly provides an injection pen with "sharps prevention." This feature allows patients to safely dispose of the needles without having the need for the patients to carry a sharps container or other suitable disposal unit with them. Additionally, this allows a patient to safely carry the pen needles between the time of use and ultimate disposal of the pen needle in a proper disposal container.

Accordingly, one aspect of the present invention provides a safety disposal cap having a top, a bottom and a side. Both the top and bottom are solid to give protection to the non-patient end of the needle. The disposal cap further contains at least one tang. The tang is disposed on the cap and is used to permanently lock with the outer cover of the pen needle. The outer cover provides protection to the patient end of the needle. Thus, both the patient and non-patient ends of the pen needle are securely covered.

### BRIEF DESCRIPTION OF THE DRAWINGS

Fig. 1 is a perspective view of the safety disposal cap assembly in accordance with the subject invention;
Fig. 2 is an exploded assembly and partial cutout view of Fig. 1;
Fig. 3 is a cross-sectional view of the cap taken along line 3-3 in Fig. 2;
Fig. 4 is the cross-sectional view in Fig. 3 with the outer cover and needle hub assembly attached thereto;
Fig. 5 is the cross-sectional view in Fig. 3 with a resealable skirt;
Fig. 6 is a perspective view of an alternate embodiment of the invention in Fig. 1;
Fig. 7 is a cross-sectional view of Fig. 6 with the safety cap partially assembled;
Fig. 8 is the view in Fig. 7 with the safety cap fully assembled;
Fig. 9 is a front view of Fig. 6;
Fig. 10 is an enlarged view of Fig. 8 showing the locking mechanism;
Fig. 11 is a bottom view of Fig. 6;
Fig. 12 is the view in Fig. 11 without the backstop;
Fig. 13 is a front view of an alternate embodiment of the present invention in Fig. 1;
Fig. 14 is a top view of Fig. 13;
Fig. 15. is a cross sectional view showing the safety cap in Fig. 13 assembled to the outer cover and needle hub assembly with the cap open; and
Fig. 16 is the view in Fig. 15 with the cap partially closed;
Fig. 17 is the view in Fig. 15 with the cap fully closed; and
Fig. 18 is an enlarged view of Fig. 17.

### DETAILED DESCRIPTION OF THE DRAWINGS

While this invention is satisfied by embodiments in many different forms, there is shown in the drawings, and herein described in detail, preferred embodiments of the invention with the understanding that the present disclosure is to be considered exemplary of the principles of the invention. It is not intended to limit the scope of the invention to these embodiments illustrated. The scope of the invention will be measured by the appended claims and their equivalents.

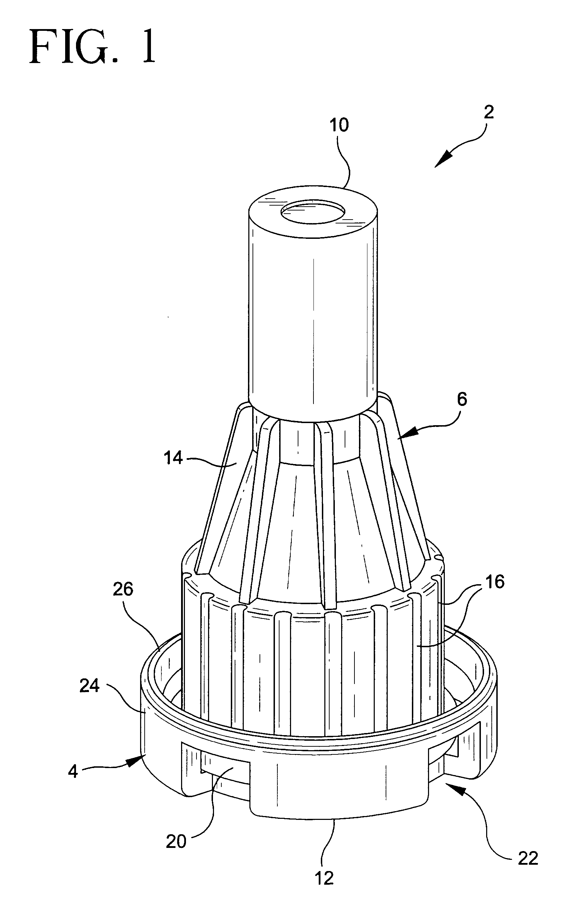

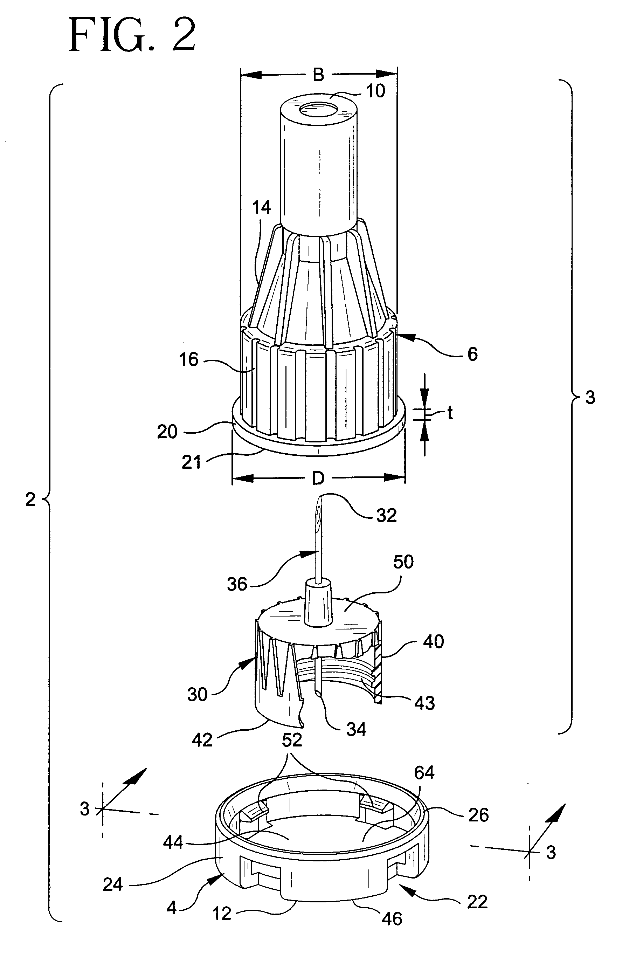

Adverting to the figures, Fig. 1 illustrates a safety disposal assembly in accordance with the subject invention generally identified by the numeral **2.** Assembly **2** has a proximal end **12** and a distal end **10.** Assembly **2** also includes a needle hub assembly **30** an outer cover **6** and a safety disposal cap **4.** When the needle hub assembly is attached to the outer cover an assembly **3** is created as shown in Fig. 2.

Outer cover **6** includes a plurality of distal ribs **14** and a plurality of proximal ribs **16.** It is within the scope of the invention that proximal ribs **16** and/or distal ribs **14** can be eliminated from the outer cover without interruption to the present invention's function. The function of the ribs are for general structural strength of the outer cover. They also assist in allowing the user to apply or assemble the pen needle onto a medicine delivery device. The outer cover has a proximal end **21** with distal end **10.** Preferably, an exposed flange **20** is disposed on proximal end **21.** However, it is within the scope of the invention that the flange may be disposed anywhere on the outer cover. Such positions, for example, would include both external and internal locations on the outer cover. However, the exposed flange is preferred on the external location of the outer cover as shown in Figs. 1-3.

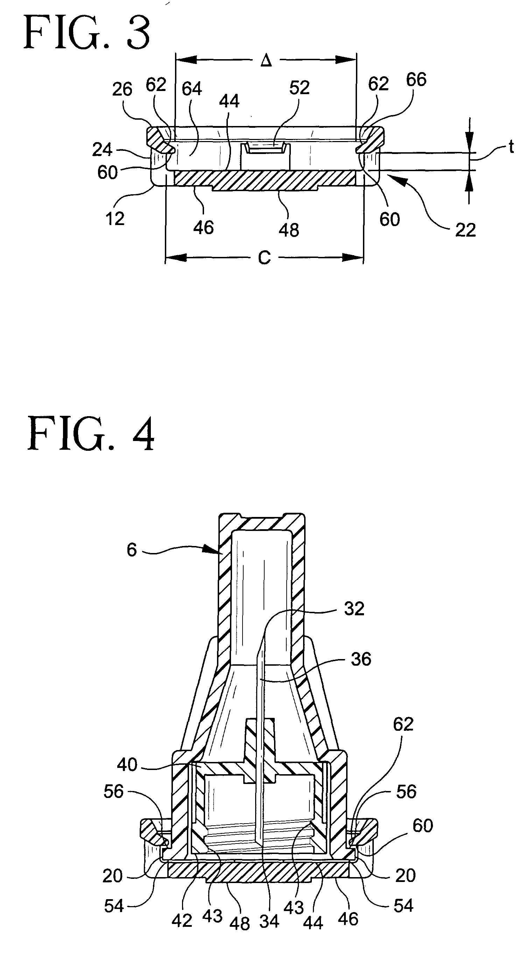

Outer cover **6,** further includes an outside diameter "**D**" and a flange thickness "**t**" as shown in Fig. 2. Preferably, when the tangs of the safety disposal cap are internally disposed, and diameter "**D**" is less than an inside diameter "**C**" of the cap as shown in Fig. 3. Additionally, the outer cover has an outside diameter "**B**" as shown in Fig. 2. Preferably, diameter "**B**" is less than a diameter "**D**". Generally, diameter "**D**" is between about 0.500" to about 0.700". In addition, the outer cover has flange thickness "**t**" preferably of between about 0.03" to about 0.05". Outside diameter "**B**", in addition, has a diameter preferably of between about 0.5" to about 0.530".

Safety disposal cap **4** includes a top **26,** a side **24,** and at least one tang **52.** The cap has a diameter "Δ", as shown in Fig. 3, which is the distance between tangs. Diameter "Δ" is preferably less than diameter "C" and less than diameter "D" of the outer cover. Preferably, tang **52** is circumferentially disposed within cap **4,** but does not have to be for the invention to function. The tangs may be disposed outside the cap such that the outer cover would lock over the cap instead of inside the cap as in the preferred embodiment.

Safety disposal cap **4** further defines a hole **64** wherein the tangs are preferably disposed. However, as previously stated, it is within the scope of this invention that tang **52** can be disposed on the outside of the cap. It is within the scope of the present invention to have tang **52** disposed on side **24** such that the safety disposal cap would be disposed within a needle hub **40** or the inside of the outer cover. The cap also has a distal opening **66,** as shown in Fig. 3, which is part of the hole. Additionally, the cap includes a length "**T**" which defines the distance from a proximal face **60** of the tang to an inner face **44** of the cap. Length "**T**" determines the thickness of the flange of the outer cover that can be locked in the cap. Thus, preferably, length "**T**" is greater than the thickness of the flange "**t**".

Safety disposal cap **4** further includes an outer face **46** and inner face **44** as shown in Figs. 1-3. Inner face **44** of safety disposal cap **4** provides protection from a proximal needle end **34** such that when the pen needle is finally disposed, both patient and non-patient ends of the needle are securely shielded to prevent any accidental needle sticks.

For needle assembly **30,** non-patient end of needle hub **40** and safety disposal cap **4** is designed such that proximal end **34** will never come in contact with inner face **44** of the safety cap. As illustrated in Fig. 2, the distance between proximal end **34** of the needle and a proximal face **42** of the pen needle hub is such that the proximal end of the needle will never come in contact with the inner face of the cap. Further included, in pen needle hub assembly **30** is a needle **36.** Needle **36** has proximal end **34** or non-patient end and a distal end **32** or patient end. Additionally included, is a needle hub **40.** Needle hub **40** has an outer roof **50** and proximal face **42** as previously described. Preferably, needle hub **40** contains a plurality of threads **43** so that the needle hub assembly can be threaded on the medication delivery device.

When the outer cover is assembled over the needle hub, proximal face **42** is preferably distal from the exposed flange of the outer cover. This positioning, as well as the positioning of the needle within the needle hub ensures that the proximal end of the needle will not come into contact with the inner face of the safety cap.

Cap **4** in addition may have a tang cutout **22** although it is not require for the present invention to function as such for the prevention of exposure proximal end **34.** Cutout 22 allows the tang to move freely and lock onto the outer cover. In the absence of cutout **22** the tangs may still freely be moved by being positioned on the cap or inner face **44.** Additionally, the tangs may freely move and may be positioned as shown in Fig. 2 without the tang cutout by having the tangs made of a flexible material. Such flexible materials include, but are not limited to, thermoplastic elastomers, thermoplastic polymers, polypropylene, polyethylene, high impact polystyrene and other rubber-modified thermoplastics.

Flange **20** of the outer cover further has a proximal side **54** and a distal side **56.** Distal end **56** is used to prevent the outer cover from being removed from the safety disposal cap. Proximal flange side **54** is used to deform the tang to allow the outer cap and pen needle hub assembly **30** to enter into hole **64.** Again, it is within the scope of the present invention for needle hub assembly **30** that outer cover **6** can be disposed outside of the safety disposal cap instead of the safety disposal cap being disposed outside of the needle pen hub and outer cover. Preferably, the safety disposal cap is disposed outside of the outer cover to prevent re-use of the enclosed needle hub.

As shown in Fig. 3, tang **52** further includes a proximal tang face **60** and a distal tang face **62.** The proximal tang face is to allow the interface between the distal flange side and proximal tang face **60** to prevent removal of the outer cover. Distal tang face **62** provides deformation of the tang when in contact with the proximal flange side **54** of the outer cover. It is within the scope of the present invention that tang **52** can be any structure known to those skilled in the art that provide a permanent fit between the safety disposal cap and the outer cover. Such structures include but are not limited to, cantilever snaps, permanent thread fits, cam configurations, self-sticking adhesives, or any other adherent structures. Preferably, tang **54** contains a distal tang face **62** having an angular shape to allow the deformation of the tang when the proximal end of the flange is forced upon the tang to complete closure with the safety disposal cap. Removal of the outer cover is prevented by the geometry of a proximal tang face **60** such that the thickness of the flange fits securely between the proximal tang face and the inner face of the cap.

Safety disposal cap **4** further includes an outer face **46.** Outer face **46** can provide indicia **48.** Indicia **48** can include safety warnings, product identification, company logos, and the like. Inner face **44** is preferably structured to prevent exposure of the proximal end **34** of needle **36.**

Fig. 3 illustrates a cross-sectional view of the present invention's safety disposal cap. A tang cutout **22** is shown below the tang. However, it is within the scope of the invention that the tang cutout can be in any position on the safety disposal cap to allow the flange of the outer cover to be locked by the tang. In Fig. 3, the flange of the outer cover protrudes outwardly from the tang cutout. Again, the flange of the outer cover is prevented from removal from the safety disposal cap by the proximal flange side **54** of the cover and proximal tang face **60.**

Alternatively, in the preferred embodiment, the cap may be molded as part of the outer cover. Such methods would include incorporating a living hinge **82** so that the lid and the flange are one piece. As with all the embodiments, the interface between the flange and the tangs are such that the flange is held in place by contact with the proximal face of the tang or the flange extension.

As shown in Fig. 4, tang **52** with proximal tang face **60** and distal tang face **62** locks the safety cap onto the outer cover sealing the needle hub assembly within and preventing exposure to the patient and non-patient ends of needle **36.** Similar to the embodiment shown in Fig. 2, proximal tang face **60** prevents the removal of flange **20** from the safety disposal cap. Preferably, as with the preferred embodiment in Fig. 3, the tangs in Fig. 4 would be circumferentially disposed within the safety disposal cap. As with the preferred embodiment, tang **52** may also be a snap ring, self-adherent adhesive, a cam mechanism, and any other locking mechanism that provides removal of the outer cover from the safety disposal cap. Additionally, as with the preferred embodiment, the safety disposal cap may be disposed inside the outer cover instead of the safety disposal cap being disposed outside the outer cover as illustrated in Fig. 4. This alternate embodiment would be made by having a flange on the inside of the outer cover in which the tangs disposed on the outside of the safety cap would lock onto in a permanent manner.

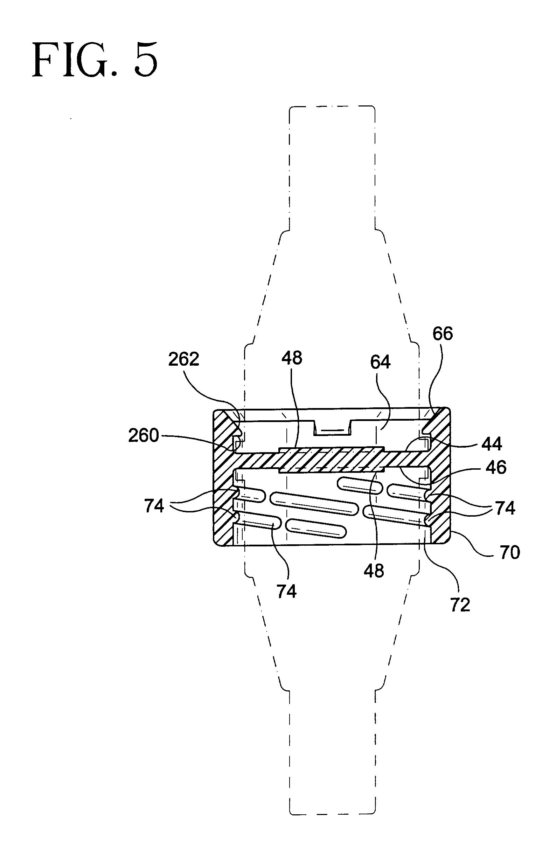

Adverting to Fig. 5, tang **52** can have many different embodiments to prevent the outer shield from being removed from the safety disposal cap. Shown is a cross-sectional view of an alternate embodiment of Fig. 3 with a resealable skirt **70.** Resealable skirt **70** contains a proximal opening **72,** and a labyrinth **74** disposed within. Labyrinth **74** could be a threaded structure, a snap-fit, or any other structure that can provide releasable mounting with the outer cover. The labyrinth would allow sterilization of the needle within the outer cover. In this alternate embodiment, tang cutout **22** is removed to provide a leak-proof and airtight seal between the safety disposal cap and the outer cover. In such an embodiment, the safety disposal cap can be used as a sterility barrier when the outer cover and the pen needle hub assembly are disposed in proximal opening **72.** After the needle has been used, the outer cover and the pen needle hub assembly are disposed in distal opening **66** where the tang permanently locks the cap over the outer cover preventing access to the needle and both its non-patient and patient ends.

After use of the needle hub, the outer cover is replaced over the needle hub and the outer cover and needle hub is placed within a distal opening **64** which is provided with a distal tang face **262** and a proximal tang face **260.** Similar to the preferred embodiment, the proximal tang face **260** prevents the flange from being removed from the safety disposal cap. Distal tang face **262** is configured to allow the flange of the outer cover to enter distal opening **64** and lock beneath proximal tang face **260.** As shown in Fig. 5, indicia **66** can be placed on both sides of either the inner or the outer face. Such indicia would note either sterility of the product or warn about the sharps contained within or other such labeling.

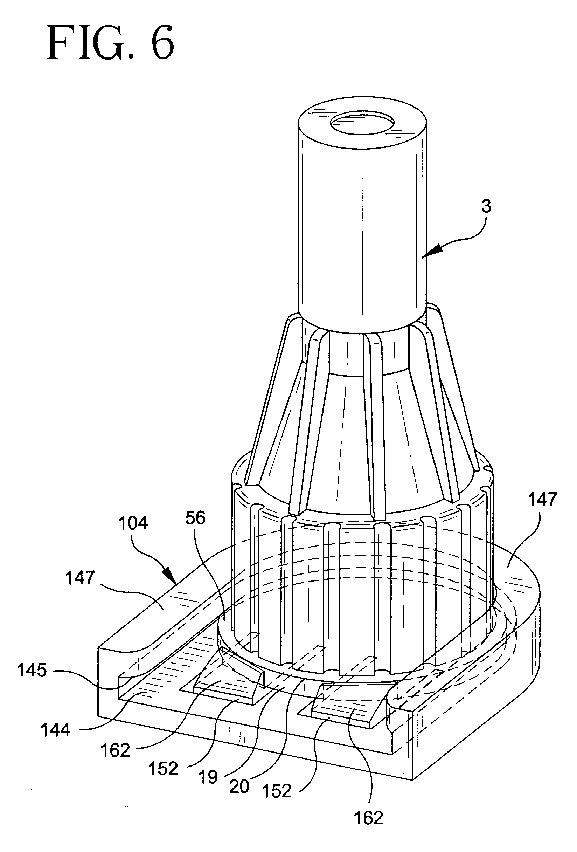

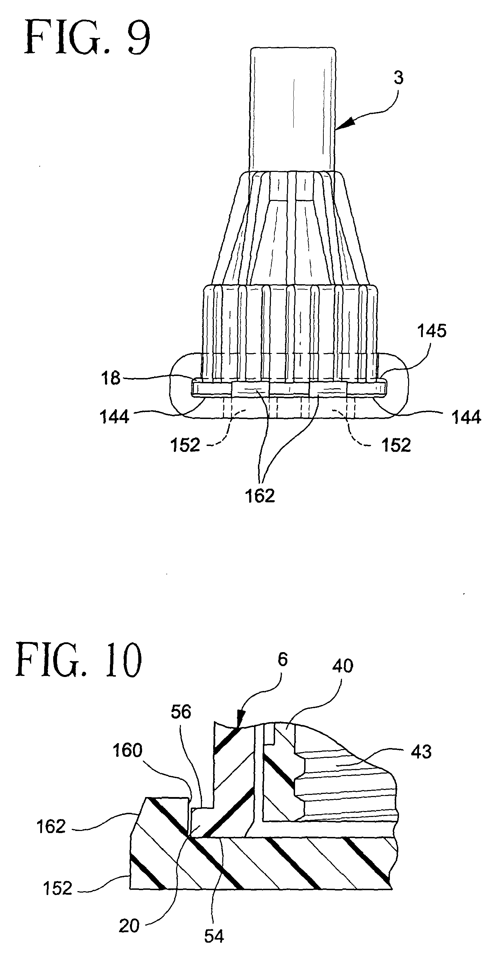

Adverting to Figs. 6-11, illustrated is another alternate embodiment of the present invention. Shown is a safety disposal cap **104** containing an outer face **146,** an inner face **144** and a distal face **145** and a top **147.** The indicia may be placed on the outer face as previously described. Cap **104** further includes an opening **143** to allow flange **20** to slidably engage into cap **104.** Upon flange **20** entering cap **104,** at least one radial tang **152** having a proximal locking face tang **160** and a distal face tang **162** is deflected by flange **20** contacting distal face **162.** In this embodiment, the flange has a side **19.** Side **19** prevents the outer cover and needle assembly from sliding out of the safety disposal cap by coming into contact with proximal face **160.**

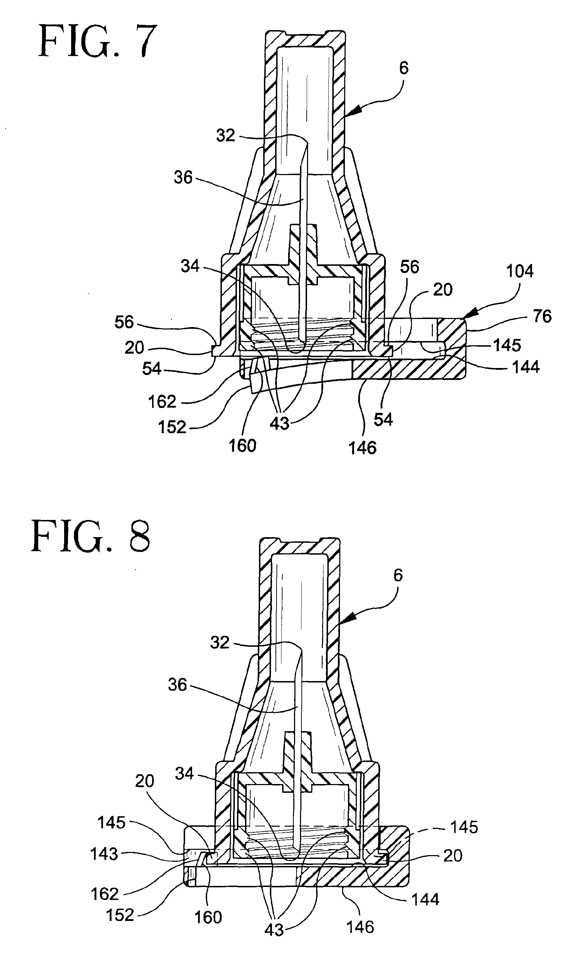

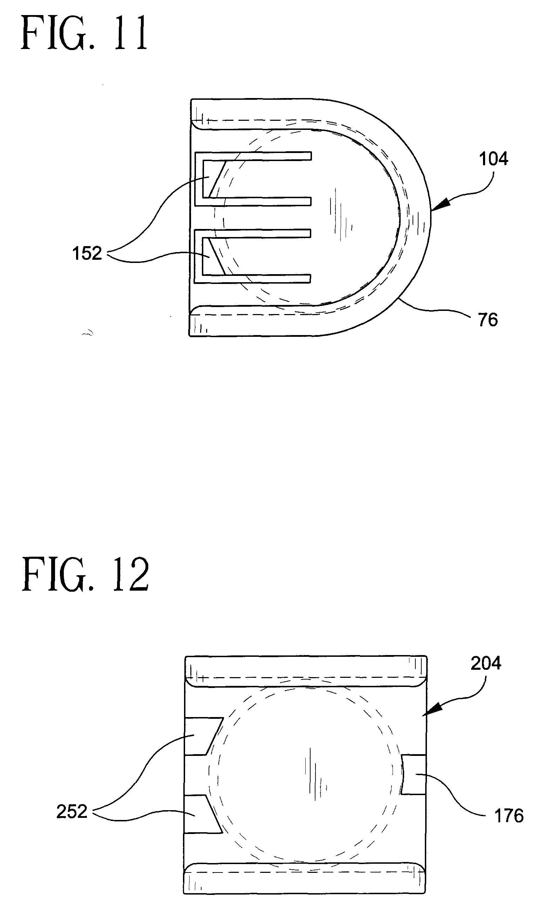

As shown in Figure 8, proximal face **160** permanently locks the outer cover and pen needle assembly in the cap by having proximal face **160** in contact with the side of flange **20.** Since both the proximal face and side of the flange are vertically flat, unlike the angular contour of distal face **162,** the outer cover and the pen needle assembly are locked into the cap. As in Fig. 7, upon the flange entering cap **104,** radial tang **152** closes such that proximal face tang **160** prevents the removal of flange **20** from the cap. Flange **20** is also prevented from being removed from the cap by a backstop **76** as shown in Fig. 11. Backstop **76** locks distal flange side **56** and proximal flange side **54** within distal face **145** and inner face **144** of cap **104.** Inner face **144** also prevents and protects the proximal end of the needle from exposure. In this embodiment, it is the inner face and distal face contact of the cap with proximal flange side **54** and distal flange side **56** that prevents the cap from being removed from the outer cover and pen needle hub. Thus, in this embodiment, the inner face of the cap, the distal face of the cap and the tang are used to lock the cap onto the outer cover.

As shown in Fig. 12, illustrated is a safety disposal cap **204** having backstop **76** replaced with a tang **176** that would prevent the flange from being removed from cap **104.** Cap **204** also has a tang **252** which functions similarly to tang **152** in Fig. 11. It is within the scope of this invention that tang **152** may also be utilized instead of tang **162.** In such an arrangement, tang **176** would be used in the preferred embodiment instead of backstop **76.** As in Figs. 6-11, the side of the flange makes contact with the proximal face of the tang thereby preventing the outer cover from being removed from the cap.

Sizing of the tangs are such that removing the safety cap is difficult without the assistance of external tools after the cap is engaged on the outer shield. However, when the cap is applied to the outer cover, the geometry of the tang is such that there provides easy assembly of the cap onto the outer cover without the necessity of external tools.

Preferably, materials of the cover and the cap consist of penetration resistant materials. Such penetration resistant materials are, for example but not limited to, polyethylene, polypropylene, impact polystyrene, polycarbonate, polymethylmethaculate, and other materials known to those well skilled in the art.

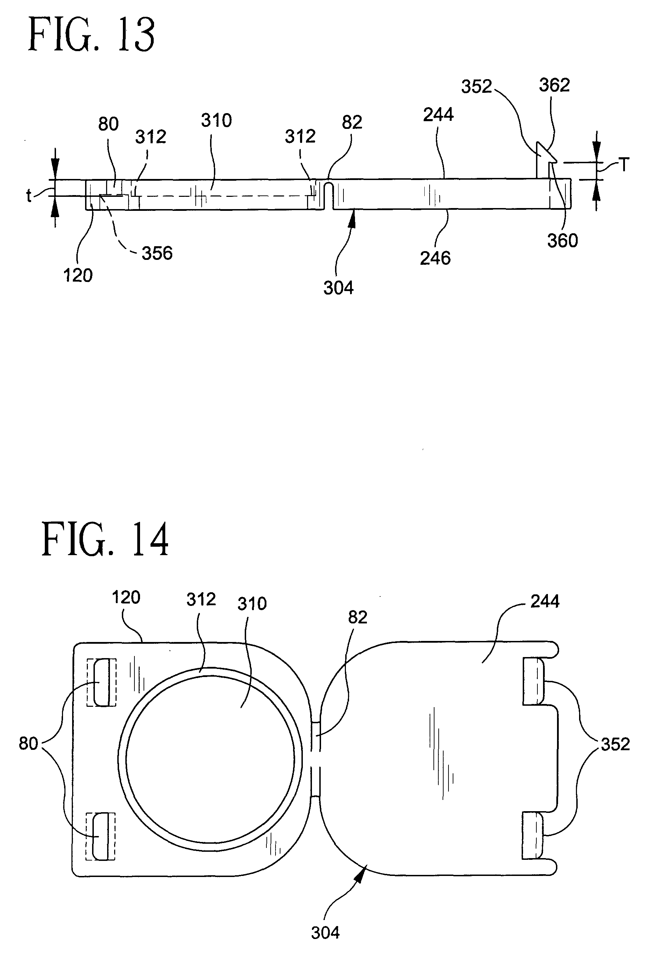

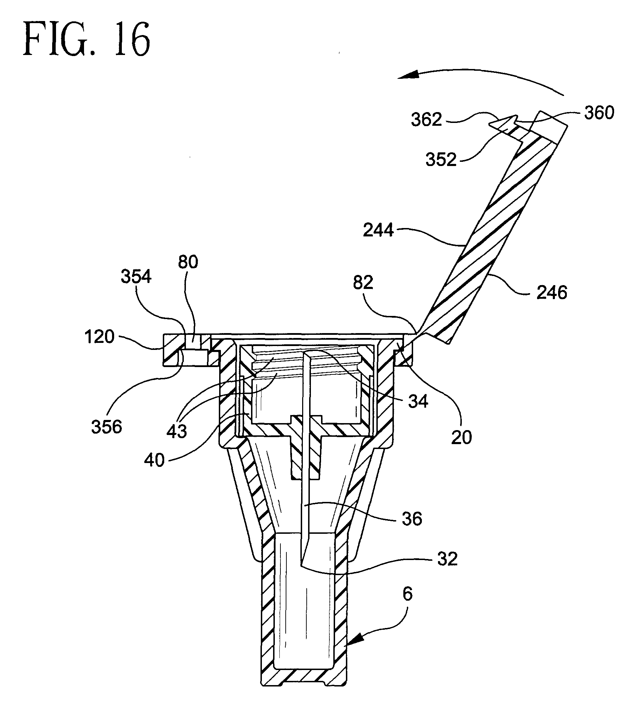

Adverting to Figs. 13-18, shown is another embodiment of the present invention shown in Fig. 1. Illustrated is a safety disposal cap **304.** Cap **304** comprises at least one tang **352** and an flange extension **120**. This cap also includes a hole **310** for the outer cover to be placed therethrough. The outer cover rests on a ledge **312** such that the flange on the outer cover rests on the ledge when place through hole **310.** Flange extension **120** further includes living hinge **82** which attaches the flange extension to safety disposal cap **304.** Safety disposal cap **304** has a proximal face **244** and an outer face **246.** Upon rotation of the cap **304** onto flange extension **120,** tang **352** locks within an engaging a hole **80** disposed on extended flange **120.** Similar to the preferred embodiment, the tangs provide a permanent lock and removal of the cap from extended flange **120** is not possible without the assistance of external tools. Furthermore, like the preferred embodiment, cap **304** provides protection against accidental needle sticks from the proximal end of the needle within the needle hub assembly.

Adverting to Fig. 13, shown is a flange thickness "**t**' " and a tang inner length "**T**' ". Preferably, length "**T**' " is greater than the distance of for thickness "**t**' ". The distance "**T**' " is defined as the distance between proximal face **244** to a proximal tang face **360** of tang **352.** A distal tang face **362** deflection of the tang as it enters the hole. The distal tang face's contoured and angular shape provides this deflection.

The alternate embodiment in Figs. 13-18 preferably functions by having cap **304** in the open position initially as shown in Fig. 13. The needle hub with the outer cover disposed thereon is then placed through cap **304** via hole **310** such that the exposed flange of the outer cover rests on flange extension **120,** preferably on ledge **312.** The flange extension then functions as an extended flange of the outer cover such that cap **304** will lock onto flange extension **120.** Proximal face **244** of cap **304** is then rotated via the living hinge so that the tang can lock into the flange extension. It is within the scope of the invention that the living hinge can be replaced by other such connecting means known by those skilled in the art. Such means include, but are not limited to, pins, loops, screws, and the like.

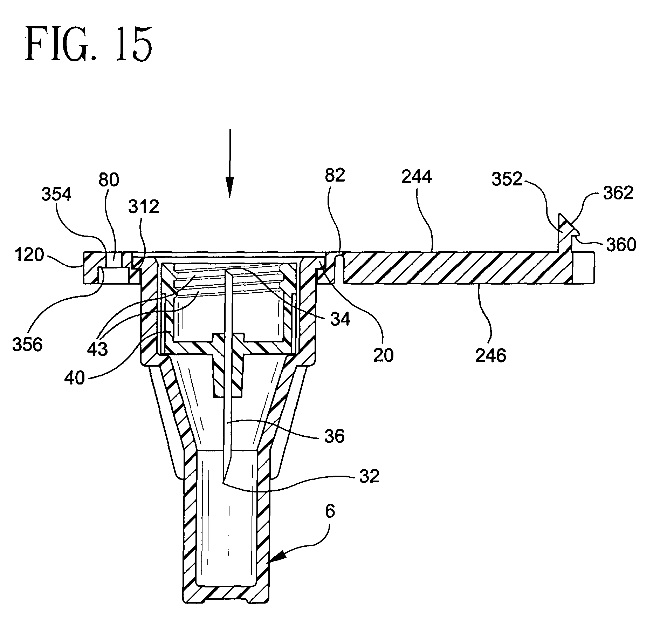

As shown in Figs. 15-17, the non-patient end of needle **36** does not come into contact with proximal face **244.** Flange extension **120** has a proximal face **354** which contacts proximal face **244** when the cap is locked. Hole **80** provides clearance to a locking ledge **356** as shown in Figs. 15-16. Ledge **356** provides locking resistance to face **360** when tang **352** enters the hole for the cap to lock. Face **362** provides the deflection of the tang in order for the tang to enter the hole.

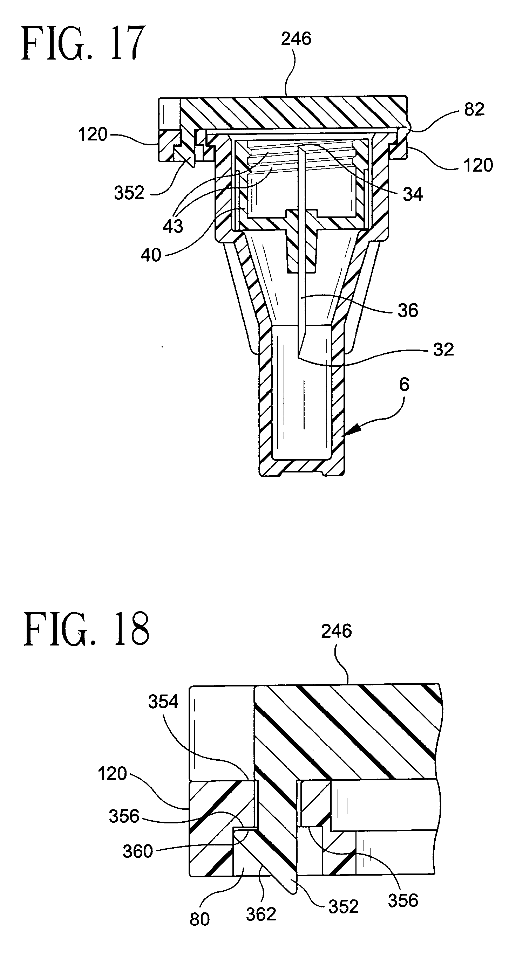

Fig. 18, shown is an enlarged cross-sectional view of Fig. 17. As with Fig 17, the cap is in the locked position. Proximal face **360** is in contact with ledge **356.** The cap is unable to open without the use of tools. This locking mechanism provides protection to the patient and non-patient end of the used needle.

The embodiments depicted in Figs. 1-18 are intended to be merely exemplary, and are not intended to pick all possible safety disposal caps. Rather, cap **4** and its alternative embodiments can be used with any pen needle assembly having the appropriate dimensioned flange thickness and diameter. The ability to provide a pen needle assembly that protects exposure to the proximal end of the needle is greatly enhanced by the present invention and its embodiments.

## Claims

1. A medical safety disposal cap assembly, comprising:
a needle hub assembly having a needle hub, and a needle disposed therethrough, said needle having a patient end and a non-patient end;
an outer cover disposed on said needle hub assembly and having a proximal end, a distal end, and an exposed flange; and
a safety disposal cap having at least one tang, said tang being deflectable and locking said flange such that said patient and said non-patient ends of said needle are lockingly protected.

2. The cap assembly of Claim 1, wherein said safety disposal cap further defines a hole, and said needle hub and said outer cover are disposed within said hole.

3. The cap assembly of Claim 2, wherein said tang is mounted within said hole and said flange is outwardly disposed on said outer cover.

4. The cap assembly of Claim 1, wherein said outer cover completely encompasses said needle hub.

5. The cap assembly of Claim 1, wherein said safety disposal cap further comprises an inner face and an outer face, and said outer cover being disposed over said patient end and said inner face being disposed over said non-patient end.

6. The cap assembly of Claim 5, wherein said outer face further includes indicia.

7. The cap assembly of Claim 1, wherein said safety disposal cap further includes a resealable skirt, said skirt having a proximal opening, and a labyrinth disposed therein.

8. The cap assembly of Claim 7, wherein said labyrinth is a threaded structure.

9. The cap assembly of Claim 8, wherein said safety disposal cap further includes an inner face and an outer face wherein said faces contain indicia.

10. The cap assembly of Claim 1, wherein said safety disposal cap further includes an inner face, a distal face, and a backstop such that said flange is locked between said faces and said backstop.
